# EUROPEAN PATENT APPLICATION

(11) **EP 1 797 843 A1**
(43) Date of publication of application: **20.06.2007**
(21) Application number: 05027410.9
(22) Date of filing: 14.12.2005
(51) Int. Cl.: A61F 2/06

(54) **Lesion specific stents, also for ostial lesions, and methods of application**

(71) Applicant: Ischinger, Thomas, 81925 München (DE)
(72) Inventor: Ischinger, Thomas, 81925 München (DE)
(74) Representative: Ruschke, Hans Edvard

(57) **Abstract**

A balloon or dilatation activated stent particularly for use in a body vessel for specific lesions, particularly in the region of the ostium of a vessel or a bifurcation featuring at Ieast two different stent characteristcs as needed for optimal stent treatment. The main portion (30) is predominantly plastically deformable and at least one end portion (20) is elastically deformable and opens to a diameter significantly larger than the diameter of the main portion thereby covering the area of a vessel bifurcation or the ostium and the adjacent vessel wall by conforming to it.

## Description

### Technical field

This invention relates to a stent and its implantation into blood vessels. More particularly it relates to a stent and an application catheter used to implant the stent into ostial lesions, vessel befurcation and lesions with specific requirements for treatment with a stent.

### Background and art

Stents are prostheses to support the lumen of hollow organs, primarily to acutely maintain the lumen of blood vessels after interventions such as balloon angioplasty and to achieve an improved long term result after such mechanical intervention. While implantation of stents into straight, non bifurcated vessel segments and vessel segments not including ostial segments pose little technical problems, implantation of stents into ostial lesions or vessel segments including the aortic ostium or into segments including the ostium of a branch vessel represents technical problems and a challenge to the operator, and carries the risk of acute and long term failure, in particular, due to imprecise placement, incomplete lesion coverage, recoil or collapse of the stent at the ostium and protrusion of the stent from the ostium with increased risk of thromboembolism, restenosis and increased technical difficulty of performing repeat catheterization, i.e. selective angiography or angioplasty.

In ostial lesions the proximal end of the stent must be placed precisely at the ostium of the artery so that the proximal end is not protruding into the aortic lumen or into the main artery from which the ostium originates. In order to avoid such risk, the stent is often advanced too far into the artery leaving the ostium itsself or ostial lesion unstented. This increases the risk of a collapse and acute or late renarrowing of the dilated yet unsupported ostium or ostial lesion. Moreover, recoil forces after dilatation procedures in an ostium are significantly higher than in non ostial areas. Also, as in all lesions and areas of a vessel it is important - and in particular when drug eluting stents are being used - to cover the lesion completely with the stent in order to achieve the desired treatment (drug) effects. Therefore the stent is chosen (as to length and localization) to extend several millimeters beyond both ends of the lesion, i.e. always longer than the lesion itsself. In the ostial lesion setting the goal is to encapsulate the target lesion with the stent material. For this purpose, however, highly conformable, nontraumatic, dense material is needed which folds around an ostium and achieves contact and high degree material coverage of the tissue surrounding the ostium, which is extending in a plane more or less perpendicular to the longitudinal axis of the vessel originating from the target ostium - rather than providing strong radial support. Similar challenges exist with stenting of side branches and vessel bifurcations. Similar challenges may alos exist for lesions in other specific locations such as severe tortuosities or lesions with specific morphologic characteristics such as thrombotic lesions.

Another major problem in the situations described above (stenting in aorto-ostial lesions, sidebranch ostial lesions and bifurcations) is the precise stent placement. The operator must rely on visual assessment during fluoroscopy and contrast injection. Contrast injections are of little value in particular for procedures in true ostial (aorto-ostial) lesions, since opacification of the target artery and the ostium are inedequate and identification of the ostial takeoff from the aorta is very limited. Visualization of side branch ostial lesions, bifurcations, in particular the beginning of the side branch ostium, are similarily difficult.

The prior art has attempted to address some of the problems described. Von Oepen (US 6048361) and Yoav Shaked (US application 20050209677) describe application catheters and modified conventional stents with a larger side hole for improved sidebranch access after stent placement. A dedicated application system for precise placement of stents into an aortic ostial lesion and a dedicated oblique stent on an application system for precise placement of stents into the ostium of side branches has been described by Ischinger (US 6682 556 B1).

Goshgarian (US application 20040260378) and others describes a dual balloon method to implant a balloon-expandable stent into an ostium, Shmulewitz (US application 20050222672) a predominantly selfexpandable ostial stent which may also employ a balloon to modify the ostial portion. All prior art proposals use either balloon expandable stent material or self-expandable stent material and complicated and unsafe ways to release the stent. Predominantly self-expandable stents are difficult to place precisely due to their shortening upon expansion or due to problematic stent release mechanisms, are of high profile (large diameter), have too low radial strength and may need post placement adjunct procedures, and may slip from the target area. Balloon expandable stents need at least two balloons to expand the ostial stent portion to a larger diameter. This involves two steps, high profile application catheters, and the coverage of the tissue surrounding the ostium by the stent material is incomplete and the stent material of a balloon expandable stent is not adequate for smooth and continous lesion encapsulation as it is only adequate for strong radial support and scaffolding.

There is no prior art that offers a practicable and safe technique to safely solve the problem of ostial stenting. The complex requirements for ostial lesion stenting combined in one device and one procedural step have not yet been met by the prior art.

The same is true for lesions in specific vessel anatomies, like sharp bends, and for lesions which contain thrombotic burden and risk of embolization of such atherothrombotic material downstream upon stent implantation. Such anatomies and such lesions with embolic risk need both an extremely high lonitudinal stent flexibility and a particularly dense and thin stent mesh structure in order to achieve nontraumatic coverage of a tortuous vessel segment or safe coverage (sealing) of a thrombotic lesion. At the same time, however, scaffolding properties, i. e. sufficient radial strength must be provided where needed along the vessel segment which is covered by the stent. Commonly, towards the ends of a stent, less scaffolding but more flexible stent material is required, while within the stenosing lesion the higher radial strength of plastically deformable strong stent struts in combination with safe protection from emboli by a thin dense mesh structure are needed. Such properties can only be satisfactorily achieved by combining distinctly different material properties and structures in one stent which then meets the individual requirements of specific lesions and anatomies optimally.

Accordingly, it is an object of the present invention to provide a radially expandable stent for implanting in a body hollow organ in the region of an ostium of a hollow organ, in particular in - but not confined to - a body vessel in the region of an ostium of a vessel and in lesions with specific requirements as described above, which avoids the disadvantages of the prior art.

This desired stent is to combine the following features:
- increased radial strength at the ostium or ostial lesion in order to withstand the increased recoil forces (collapse) of the ostium,
- use of dilatation expandable stent techniques for ease and safety of stent delivery,
- precise placement even with limited control by contrast injections,
- low profile/cross section and high flexibility,
- potential to be firmly seated in the ostium without the risk of displacement in neither direction, particularly not towards the aorta (i. e. without the effect of self-displacement in axial direction),
- non traumatic coverage of lesions with embolic risks by ultra-thin, dense and conformable stent material in order to achieve safe sealing of the lesion prior to scaffolding,
- complete coverage (encapsulation) of an ostial lesion by ultrathin and dense stent material which conforms to the larger diameter of the main artery, or, in case of an aortic ostial lesion, self orients and extends to the aortic wall, in a plane more or less perpendicular to the longitudinal axis of the vessel carrying the ostial target lesion. Thereby no stent material is protruding freely into the lumen and the blood flow. Instead, the lesion is fully encapsulated by the stent and the ostial portion of the stent is in contact with the adjacent anatomic structures like adjacent aortic or main vessel wall.

These requirements can not be met by one single stent or one single stent material but rather by the combination of two distinctly different stent material properties. Basically, by the use of two different stent properties on top of each other the extreme potentials of each material can be used and combined in a way that the different requirements of an ostial target lesion or other specific lesion requirements can be appropriately met. In the stent of the present invention particularly for ostial use this means that an ultrathin, highly conformable self expandable material with a dense material structure is used for ostial encapsulation and a dilatation expandable plastically deformable scaffolding material with sufficient radial strength is used for the main and more distal segment and a region of overlap is created of both stent materials in order to have an interaction of both stent material properties along an area with the need for both increased radial strength and increased density of stent structure and higher tissue coverage.

The mere arrangement of different stent material properties or structures in an axial sequence fails to use the benefit of the interaction of two overlapping distinctly different material properties.

The present invention offers a unique solution to the technical problems as described above in the ostial, aorto-ostial and bifurcation setting as well as in specific lesion requirements, but not confined to those areas.

### Summary of invention

One embodiment of the present invention comprises a balloon- activated (or activated by other dilatation means) radially expandable cylindrical stent assembly which has an essentially plastically deformable first cylindrical stent extending to the distal end of the stent assembly and forming a distal opening, and a second essentially elastically deformable stent forming a proximal opening, wherein the first and the second stent form a segment of overlap located between the proximal and distal ends of the said stent assembly. The proximal end portion of the elastically deformable (second) stent features the potential to expand - if unconstrained by a blood vessel wall or by a mechanical means - to a cone- or trumpet-like shape. The proximal elastically deformable portion of the stent is positioned in total or at least partially proximal to the target ostium and has the ability to open trumpet-like, in such a way, that the proximal stent portion approaches the surrounding tissue and comes in contact with it, thereby creating a plane of stent material which is more or less perpendicular to the longitudinal axis of the stent assembly inside the target vessel and inside the ostium.

The stent assembly of this invention exhibits three distincly different properties:
1) plastic deformability and scaffolding property;
2) elastic deformability and conformability by self-expansion of at least one end segment;
3) increased radial strength, increased material density of the stent and increased sealing ability of the lesion in the area of the overlap of the first and second stents.

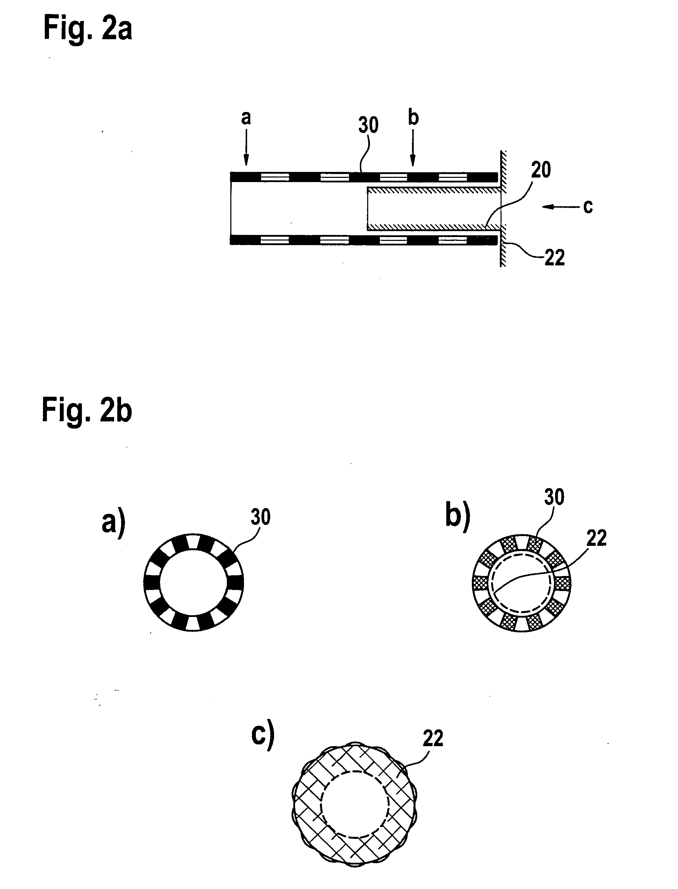

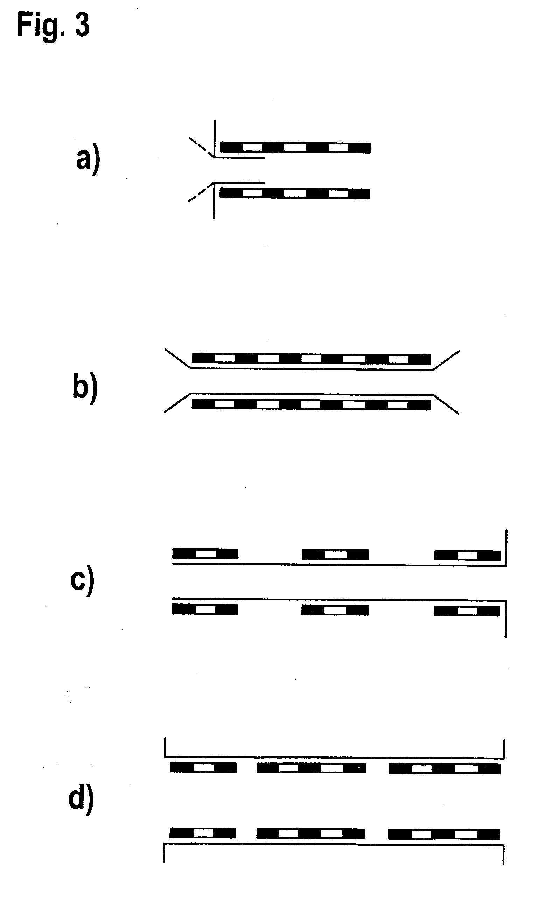

One embodiment that incorporates these different stent properties incorporates at least one elastically deformable tubular stent inserted inside at least one plastically deformable tubular stent with any given length of overlap. In a preferred embodiment, such stents are physically connected to form a dual stent assembly in the form of one single stent. At least one end portion of the dual stent device is formed by the elastically deformable thin, dense and conformable stent material (see embodiments of Figs. 3a and 3b). For aorto-ostial lesions or other ostial lesions this may preferably be the proximal end of the dual stent assemby (see Fig. 2a).

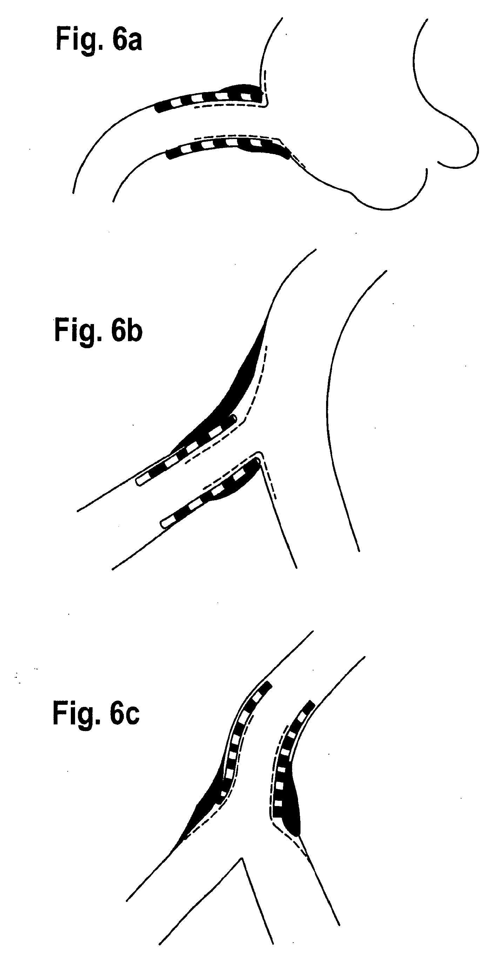

In another embodiment, e. g. for use in lesions at bifurcations or proximal to bifurcations it is the distal end of the stent assembly which is formed by the selfexpanding and elastically deformable material alone (see embodiments of Figs. 3a or 6c).

In yet other embodiments, the region of overlap essentially extends over the entire length of the plastially deformable stent (see embodiments of Figs. 3b or 3c or 3d).

In still other embodiments, muliple regions of longitudinal material overlap may be created which may alternate with longitudinal segments wherein material is used alone (see Fig. 3c). Multiple variations of this dual stent concept are conceivable.

The overlapping portions also serve as constraining and retaining means for the elastically deformable stent (commonly referred to as self expandable stent) on the application/delivery catheter. In the expanded state the overlap serves as reinforcement means for radial strength of the stent necessary at the site of the lesion and at the ostium of blood vessels. Moreover, it serves as a segment of increased stent material density for improved coverage of the lesion, for prevention of plaque protrusion and embolization through stent struts as known from prior art balloon-expandable stents, and for more uniform and versatile drug elution capacity in case of drug coating of the stent.

The crossectional plane of the proximal end of the balloon-expandable plastically deformable tubular stent may be oblique and not perpendicular to the axis of the body of the stent, thereby creating a long and a short short side of the balloon-expandable stent. This end configuration would permit the trumpet like elastically expandable segment protruding from the inside of the balloon-expandable stent to conform better to any ostial anatomy in cases where the ostial plane is not perpendicular to the longitudinal axis of the target vessel arising from such ostium.

The dual stent assembly of the present invention is mounted on an expansion means (such as a balloon) on a delivery catheter. Expansion of the expansion means expands the plastically deformable portion of the dual stent assembly to embed it into the vessel wall. This enables the selfexpandable stent to increase its diameter along the overlapping segment accordingly. The self-expanding segment has in its expanded state a fully open trumpet-like proximal end portion which has the tendency, due to its preformed shape-memory characteristics, to orient itself towards the ostium as it expands fully and retracts to the adjacent vessel wall, e. g. of the aorta, if used in aorto-ostial lesions (see Fig. 6a). The elastically deformable stent with its trumpet like portion must preferably be made of shape memory metal or metal alloy or other shape memory material.

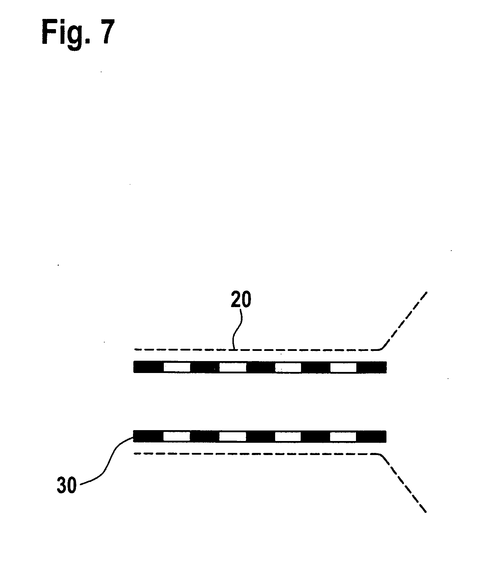

In other embodiments (Fig. 3d or Fig. 7) the positions of the dual stent assembly are inverted: a plastically deformable stent is positioned inside a self expandable stent and both stents are firmly connected to form one single stent assembly. In these embodiments the inner stent forces the outer stent (self-expandable) to follow along the area of stent overlap and stent fixation. These versions result in a particularly smooth outer surface of the dual stent assembly avoiding sudden changes of the outer surface of the dual stent assembly (Fig. 3d).

The dual stent devices as described above may be retained on the expansion balloon by crimping the plastically deformable or balloonexpandable stent on the balloon, as known in the art, thereby holding the elastically deformable stent in place and at least partially constrained. The proximally protruding trumpet-like portion of the self-expandable stent is constrained on the balloon by ties connected to the balloon or ties or adhesion forces connected to the stent struts itself or by a sheath (tube) surrounding the self-extending protruding stent portion. If such sheath (tube) is used, in order to release the self-expandable stent portion, the sheath (tube) is wirthdrawn by the operator as is known in the art.

Ties as described in the preceeding paragraph may be absorbable or nonabsorbable and may be an intergral part of the proximal stent portion. Such ties or links or other adhesion means between stent struts or between application catheter or dilatation means and stent will break or release upon balloon expansion or activation of other expansion means. Release of the proximal stent portion may be activated by other physical means transmitted through or along the delivery catheter as activated by the operator.

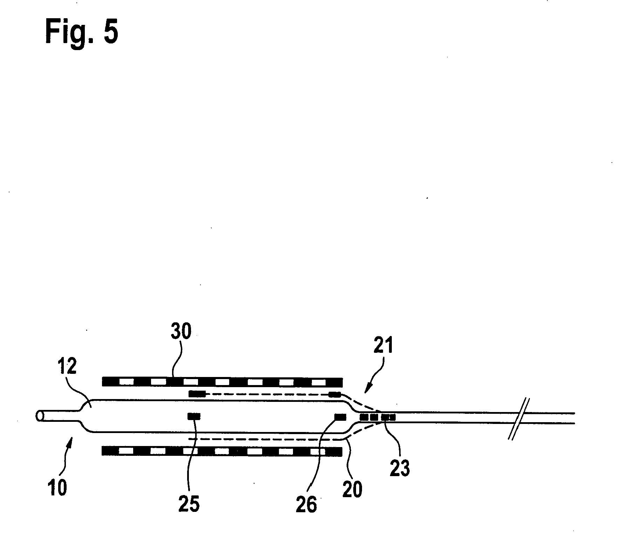

In another embodiment, such as shown in Fig. 5, the proximal trumpet-like self expandable portion is held constrained by magnetic forces between the stent elements or between the stent elements and the application catheter like a magnetic band or a specific element (like a wire) associated with the application catheter or used independently from the application catheter. The magnetic forces lose their holding force upon stent expansion or upon other activation means. The trumpet-like self expandable proximal stent portion can also be restrained on the delivery catheter by use of a bistable stent construction of the elastically deformable stent. Such a bistable tubular stent construction has two separate stable positions, namely one first position with a small diameter and a second stable position with a larger diameter, wherein the stent is moved from the first stable position to the second stable position by mechanical expansion such as by the balloon catheter.

All embodiments described for a stent assembly with a selfexpanding proximal trumpet like portion may be used similarly for embodiments with multiple regions of stent material overlap, in particular for the embodiments with the distal end portion of the stent assembly being formed by an elastically deformable stent or with both end portions being formed by elastically deformable trumpet like shaped segments.

It is also possible to provide a self-expandable elastically deformable end portion which is not restrained by overlapping materials, instead it may be retained and constrained by folding the endportion inwards so that it comes to lie in between dilatation means, e.g. balloon, and the stent assembly.

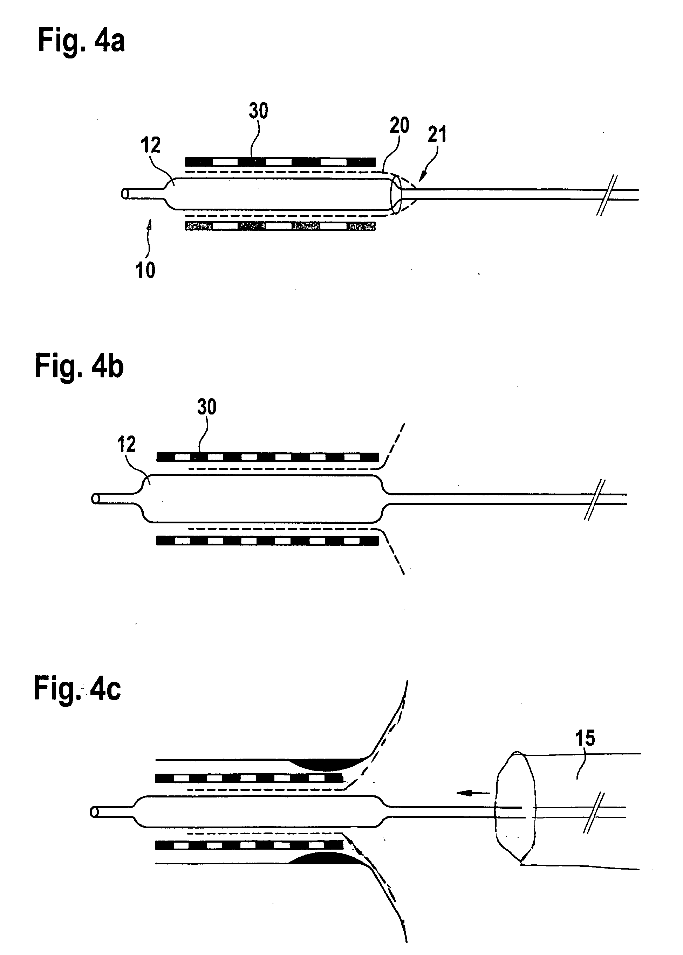

However, the proximal trumpet like elastically deformable portion may remain unconstrained as the stent assembly is introduced through the guide catheter. It may be partially constrained by the guide catheter (or other catheters through which it is advanced) and selfexpand as the assembly exits from the distal end of the guide catheter. The stent may then be introduced into the aorto-ostial lesion and self anchor and position due to the partially expanded proximal end which prevents further advancement (Fig. 4c). Activation of the stent assembly as described above will then achieve full expansion and complete the ostial stent implantation procedure.

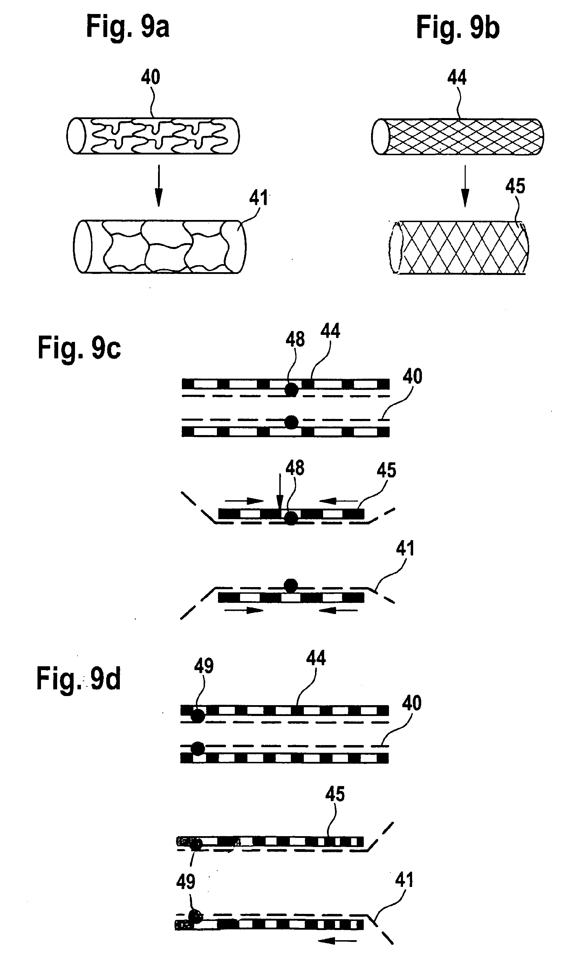

In another embodiment of the tubular stent assembly, the two distinctly different stent structures and properties of the first stent (plastically deformable and balloonexpandable) and the second stent (elastically deformable and self expandable) as described earlier for this invention are additionally characterized by their distinctly different abilities to maintain their longitudinal dimensions (lengths of the stents) upon radial expansion. In a preferred embodiment, the plastically deformable outer stent is overlapping the elastically deformable inner stent in its entire length. The outer stent foreshortens (shrinks) longitudinally upon radial expansion while the inner stent essentially maintains its length after radial expansion. Thereby the inner stent is partially freed from the outer stent. Depending on the location of the connection points of the inner with the outer stent, the process of foreshortening of the foreshortening outer stent occurs bidirectionally towards the middle of the stent - if the connecting points are arranged in the mid area of the stent assembly - or unidirectionally towards one end of the stent assembly - if the connecting points are arranged in the area of either end portion of the stent assembly (see embodiments of Fig 9c and Fig 9d). Thus, by using the different potential of the first and second stent to shrink longitudinally, both end portions or only one end portion of the elastically deformable inner stent can be released by a one step expansion procedure of the dual stent assembly so that at least one elastically deformable end portion of the seond stent can assume its preformed trumpet like shape.

The different abilities of the first and second stent to maintain the axial dimension (length) of the stent of its unexpanded state during expansion (or the different potential of the first and second stent to foreshorten their axial length upon radial expansion) is achieved by choosing a stent structure (architecture) of the first stent which is distinctly different from the structure of the second stent: the structure of the first stent (plastically deformable, outer stent) essentially and preferably consists of diamond or rhomboid shaped cells which are connected to each other in radial and axial extension thus forming the tubular wall structure of said first stent. This tubular stent structure as known per se has the ability to foreshorten longitudinally upon radial expansion (see Fig 9b). The structure of the second stent as also known per se (elastically deformable, inner stent) essentially and preferably consists of predominantly radially extending sinusoidal elements which alternate in the longitudinal axis with predominantly longitudinally extending sinusoidal elements. This tubular stent structure has the ability to more or less maintain or even increase by use of longitudinally self-expandable structural elements its length of the unexpanded state after radial expansion of the stent (see Fig 9a). Many different variations of such stent wall architectures are known.

It is possible to cover at least one stent of the dual stent assembly with a sleeve of fabric material or the like or plastic material such as PTFE, as used in so-called covered stents and as known in the art. The purpose of such sleeve is to improve the sealing ability of the stent and / or to improve the ability of one stent to slide relative to the other as described in more detail futher below in connection with Figures 9a and 9d.

The structure of the elastically deformable stent segment may be significantly thinner and denser and more flexible, with significantly lower radial force, than the plastically deformable stent structure, since it does not have to carry any load or withstand recoil of the vessel as it is used in an overlapping combination with the plastically deformable stent as described above. Therefore extremely thin stent struts or wire meshes forming a stent can be used, which lend extreme flexibility, low profile, dense mesh structure and adaptability to anatomic configurations to the elastically deformable stent segments, which can be arranged in varying lengths or locations of overlap in order to create a highly lesion specific and anatomy specific stent, for which exists a particular need in (aorto) ostial lesion, but also for other targets in the diseased vasculature, such as long lesions with varying plaque structure, varying diameters along a lesion, lesions with high risk of embolization and in difficult anatomies, like severe vessel curves (tortuosity).

In a method or practicing the concept of the present invention the inner stent, preferably the self-expandable and elastically deformable stent is not physically connected with the outer stent (balloon-expandable stent) and not forming a dual stent device as one unitary stent: The outer stent is implanted first in order to create the basis and the scaffolding for the inner stent which is slideably introduced into the pre-implanted and expanded outer stent and then released inside the outer stent. In this case, the outer stent was implanted by using known techniques for precise stent placement into the ostium. The outer stent then serves as radio-opaque landmark for precise placement of the thin and conformable inner stent which achieves ostial encapsulation by unfolding of its proximal trumpet-like end portion. In this case, the two stents of the dual stent assembly are implanted in a timely sequential procedure.

The novel features of the stent and its application which are considered characteristic for the present invention are set forth in the claims. The invention itself, both as to its construction and operation together with additional objects and advantages thereof are best understood from the following description of specific embodiments when read in connection with the accompanying drawings.

### Brief description of the drawings

Fig 1a is a schematic view showing the lesion in a true ostium originating from the aorta;
Fig 1b is a similar view showing the same situation at some unspecified main vessel;
Fig 2a is a view of one embodiment of the invention wherein the balloon expandable stent segment surrounds the self expandable stent segment. The two segments are firmly connected and exhibit the three desired properties: balloon expandable stent property, reinforcement (overlap), and the protruding proximal trumpet-like selfexpandable portion.
Fig.2b shows two different cross sections and one view as indicated by arrows, a, b, and c in Fig. 2a, namely two cross sections at the locations "a" and "b" in Fig. 2a and the end view of the dual stent assembly in Fig. 2a as indicated by arrow ,,c".
Fig 3 shows different embodiments of the dual stent assembly of the present invention.
Figs. 4a, b, c show different stages of the placement of one preferred embodiment of the present invention.
Fig. 5 shows another embodiment of the present invention.
Figs. 6a, b, c show different placement situations of the dual stent arrangement of the present invention.
Fig. 7 shows the general structure of a dual stent arrangement having an "inverted" stent structure.
Figs. 8a, b show the dual stent arrangement of the present invention in which the ostium runs obliquely relative to the side branch vessel.
Figs. 9a, b, c, d show the dual stent arrangement of the present invention before and after expansion using stent structures and materials of different degrees of shortening expansion

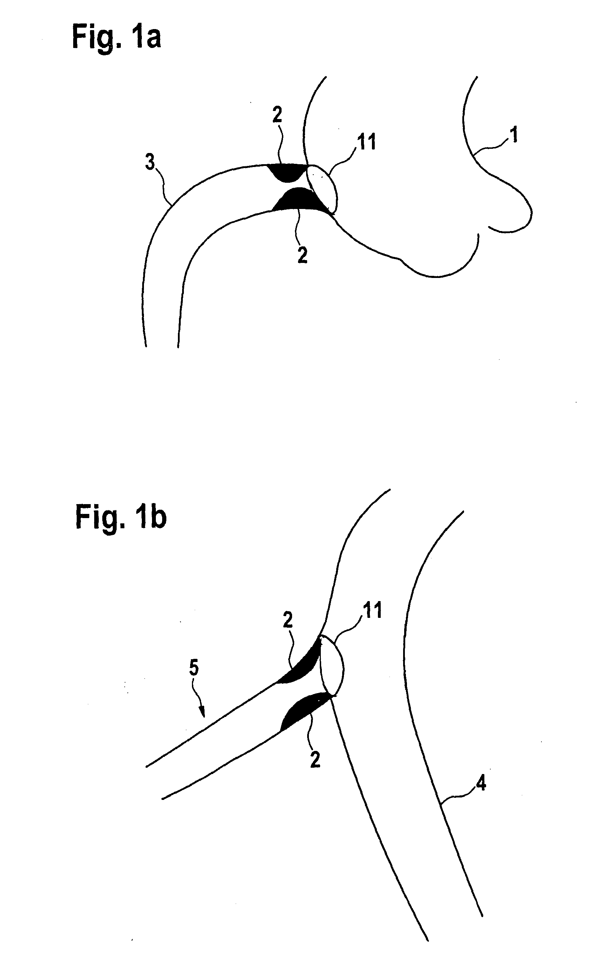

Fig. 1a shows an aorto-ostial lesion 2 at the proximal origin (ostium) 11 of the target vessel 3 originating from the aorta 1.

Fig. 1B shows a lesion 2 at the ostium 11 of a side branch 5 originating from a main vessel 4.

Fig. 2a shows a longitudinal cross-section of a typical preferred embodiment of the present invention. The inner stent 20 is a self-expandable elastically deformable stent having small gap sizes, namely a stent with a dense mesh structure and ultra-thin stent struts featuring highly conformable material, e. g. ultra-thin Nitinol mesh structure as known in the art per se. It is preferably composed of a shape-memory alloy or other shape-memory material including polymer fibers as known in the art and in its final use position in a blood vessel or the like it will assume the position/shape as shown in Fig. 2a. As shown in the drawing, the axially protruding outer portion 22 of the inner stent 20, namely the portion not surrounded by the outer stent 30, has unfolded so as to essentially lie in a plane more or less perpendicular to the longitudinal axis of the outer stent 30 and of the inner stent portion inside the outer stent 30. The outer stent is a balloon expandable plastically deformable stent having large gap sizes and a less dense mesh structure with thick stent struts featuring high radial strength. Arrows a and b in Fig. 2a indicate the location of the cross-sections a and b as shown in Fig. 2b and arrow c in Fig. 2a indicates the viewing direction of picture c in Fig. 2b: Picture c shows the right-hand front end view of Fig. 2a in which the external portion of the inner stent 20 (not inside the outer stent 30) in Fig. 2a has unfolded (due to its shape-memory characteristics) thus forming a more or less cone-like stent surface 22 lying more or less perpendicular to the longitudinal main axis of the two coaxial stent portions 30 and 20. Since the external part of the ultra-thin mesh structure of the elastically deformable inner stent structure is not being held back by the outer stent as shown in Fig. 2a (and in the front view of picture c of Fig. 2b), this external / proximal part of the dual stent structure of Fig. 2a forms a stent layer which runs more or less perpendicular to the longitudinal axis of the dual stent device, thus forming the possibility to encapsulate an ostial lesion as shown in Figs. 1a and 1b. Figures 6a and 6b show the final placement of the dual stent structure in two examples of ostial anatomies.

Fig. 3 shows different possible embodiments of the dual stent structure of Fig. 2a. Fig. 3a shows a cross-section of a dual stent structure having an internal stent section at the distal end, which is shown unfolded at different degrees. This embodiment is particularly useful in bifurcations.

Fig 3b shows a long outer one-piece stent having an inner one-piece stent which protrudes in a axial direction both at the proximal and at the distal end and forms such angled surfaces at both ends as described in more detail in connections with Fig. 2a. This embodiment is particularly useful for thrombus-rich lesions with risk of embolization.

Fig. 3c shows the same type inner stent as in Fig. 3b with an internal section at the proximal end only, however, the outer stent shows several separate length-wise portions for increased longitudinal flexibility of the stent assembly as particularly useful in extreme vessel tortuosity.

Fig. 3d shows a dual sent structure having an inner and an outer stent of the same type as described above, except both the outer and the inner stents are firmly connected and arranged in an inverted position: The ultra-thin dense elastically deformable mesh stent is now forming the outer stent structure and the scaffolding ballon-expandable stent is forming the inner stent structure. This dual stent device features a particularly smooth outer surface with high material coverage of the vessel wall (see also Fig. 7).

Fig. 4a shows an example of a dual stent structure of the present invention of Fig. 2a, except arranged on an application catheter 10 before complete release of the stent structure 20, 30 by way of dilatation of the balloon 12 of the catheter. As shown in Fig. 4a, the inner stent 20 as represented by dashed lines has not yet unfolded in the area protruding axially from the distal end of the outer stent 30 because it is still being held down/ together by a constraining tie as at 21, or similar arrangement - as also shown in the Fig. 5 embodiment, see there the magnetic elements serving to retain the corresponding parts of the inner stent 20 which otherwise would unfold as described in connection with fig. 2a. Once the catheter/balloon/dual stent arrangement of Fig. 4a is positioned properly in place at the ostial lesion (using known techniques as disclosed by Ischinger in his WO 99/03426), the balloon 12 is caused to expand in a manner well-known per se, causing the outer balloon expandable (plastically deformable) stent 30 to expand, as a result of which the inner self-expandable (elastically deformable) ultra-thin inner stent 20 will follow and the constraining tie at 21 of the protruding proximal portion of the inner stent 20 is broken or moved by the expanding balloon 12, thus releasing said protruding proximal end at 21 and allowing it to unfold to a position as shown in Fig. 2A, and as also shown in the view of picture c of Fig. 2b. The more or less unfolded position of the protruding end of the inner stent is also shown in Fig. 4b.

The constraining means or tie as indicated at 21 is known in the art and usually consists of a sheath surrounding that particular (proximal) area of the elasticallly deformable stent, which sheath structure will either be split or broken upon balloon expansion or can be withdrawn axially by the operator.

Fig. 4c shows a different alternative of introducing the dual stent structure of the invention into the target area: In this embodiment the proximal protruding end of the inner elastically deformable stent 20 is not constrained as shown in Fig. 4a. Instead, the dual stent structure without any restraining means for the unfoldable proximal end of the inner stent is advanced through the guiding catheter 15 all the way to the target area, at which time the dual stent structure is pushed out of the guiding catheter 15 into the lesion area and the proximal end unfolds and defines a self-positioning stop mechanism at the ostium.

Fig. 5 shows a similar application catheter 10 with a balloon 12 as a stent expansion means for a very similar dual stent arrangement 20, 30. There are two radio-opaque markers 25, 26 on the application catheter /balloon identifying the axial position of the distal end of the inner stent 20 and of the axial position of the proximal end of the outer stent, thus making the axial positions thereof making them clearly visible on a X-ray screen or on a similar picture producing system assisting the operator in the context of placing the dual stent arrangement at the proper location.

Fig. 5 also shows a slightly different (than in Fig. 4a) constraining arrangement at 21 for the protruding portion of the inner self-expandable stent 20: This can be accomplished e. g. by a magnetic retaining system retaining the proximally protruding elastically deformable portion of the inner stent until it is ready to unfold to a position as shown in Fig. 4b, for example. The magnetic retaining system is comprised of some magnetic material at the proximal end of the inner stent 20 and of some magnets 23 on the proximal end of the balloon or on the neighbouring hose/catheter section as shown in Fig. 5. Once the expansion/ balloon means is activated, the magnetic forces will not suffice any more to retain the proximal stent end so that the elastic self expansion/unfolding occurs at this time.

Fig. 6a shows the dual stent structure of the present invention implanted in a situation as shown in Fig. 1a.

Fig. 6b shows the dual stent structure of the present invention implanted in a situation as shown in Fig. 1b.

Fig. 6c shows the dual stent assembly of the present invention in a similar situation as shown in Fig. 6b, however, the lesion is approached from the main vessel 4 proximal to the bifurcation. In this situation a dual stent assembly as shown in Figs. 3a or 3b would be preferably used.

Fig. 7 shows an inverted arrangement of the dual stent structure in which the outer and inner stents have exchanged positions to form a "reverse arrangement" of the stents, which requires that both stents 20, 30 are firmly connected to each other such as by glueing or welding or partly intertwining or the like along the axial overlap: In this inverted situation the stent 20 is again a dense mesh ultra-thin stent and elastically deformable and self-expandable but this time it is located on the outside of the dual stent structure. The other stent 30 is balloon-expandable and plastically deformable and provides strong scaffolding/supporting properties but it is located on the inside. The important aspect again is that the axially protruding part of the thin elastically deformable self-expanding (now outer) stent can unfold (when released) to a position as shown e. g. in Fig. 2a or in Fig. 7.

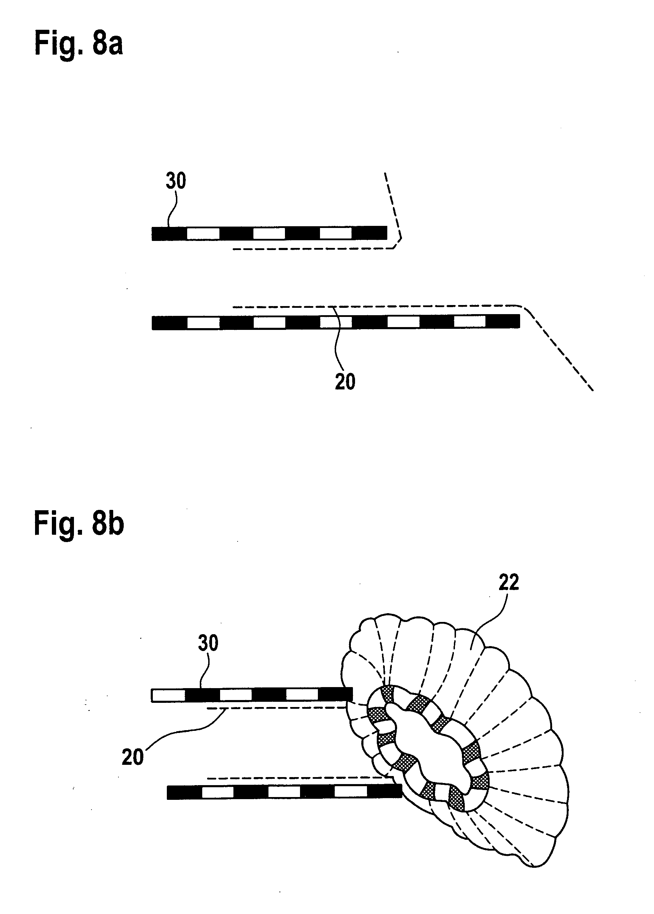

The embodiment in Fig. 8a shows a different version of the stent device of Fig. 2a in which the stronger/thicker plastically deformable stent 30 is on the outside and forms an oblique cross-sectional plane at the proximal stent end of the balloonexpandable stent. The inner stent 20 is again formed of elastically deformable thin mesh stent material. As a result thereof the cross-sectional plane of the proximal end (opening) of the dual stent arrangement is non-perpendicular, i. e. obliquely to the longitudinal axis of the main portion of the coaxial dual stent arrangement.

Fig. 8b is a partly perspective view of the stent of Fig. 8a, at least of the proximal front end therof. As shown therein, the front (proximal) end of the self-expandable elastically deformable inner stent 20 has unfolded and forms a surface or area for placement at the ostium of an ostial lesion or a side-branch lesion. The unfolded surface 22 defines a "plane" arranged somewhat oblique relative to the main longitudinal axis of the dual stent main body.

Figure 9a shows a tubular stent structure (40) in the unexpanded state which consists of predominantly radially extending sinusoidal elements, which alternate in the longitudinal axis with predominantly longitudinally extending sinusoidal elements, thus forming the wall of the tubular stent. Radial expansion of the tubular stent (40) results in a modification in the configuration of the structure of the stent wall (41), the length of this tubular stent remains essentially unchanged after expansion.

Figure 9b shows a tubular stent structure in the unexpanded state (44) which consists of an essentially rhomboid or diamond like cellular structure which extends uniformly radially and axially to form the wall of this tubular stent. Upon mechanical radial expansion, this stent foreshortens (shrinks) significantly (45) longitudinally, usually from both ends towards the center of the longitudinal axis (45).

Figs. 9c and 9d show two variations of a dual stent assembly as composed of two different stents as explained in connection with Figs. 9a and 9b, respectively.

Figure 9c shows a longitudinal crossection of a dual stent assembly using a first and a second stent structure with distinctly different potentials to foreshorten longitudinally (shrink longitudinally).In the unexpanded state of the stent assembly the first stent (outer stent, plastically deformable, balloonexpandable)(44) overlaps the second stent (inner stent, elastically deformable, self expandable)(40) over the entire length. In this example, the first and the second stent are connected at connection points (48) arranged around the middle (center) of the longitudinal axis of the stent assembly. After expansion of the stent assembly, the outer stent has foreshortened significantly (45) bidirectionally from both ends as compared to its initial unexpanded state (44), while the inner stent after expansion (41) has essentially maintained its initial length of the unexpanded state (40).

Figure 9d shows a longitudinal crossection of an other example of the dual stent assembly of Figure 9c in the unexpanded state with the inner stent (40) and the outer stent (44) being connected at connecting points (49)arranged around one end portion of the dual stent assembly. After expansion of the dual stent assembly, the outer stent (45) has significantly forshortened in an unidirectional way, i.e. predominantly towards the connecting points (49) on one end portion of the dual stent assembly.

### Reference numerals

- 1: aorta
- 2: ostial lesion
- 3: target vessel
- 4: main vessel
- 5: side branch
- 10: application catheter
- 11: Ostium
- 12: balloon
- 15: guide catheter
- 20: inner stent
- 21: constraining tie
- 22: outer portion/ring like stent surface
- 25: radio-opaque marker
- 26: radio-opaque marker
- 30: outer stent
- 40: stent unexpanded with stable axial length
- 41: same as 40, expanded
- 44: stent, unexpanded, foreshortening upon expansion
- 45: same as 44, expanded
- 48: central connection point
- 49: excentric connection point
- a, b, c:: arrows in Fig. 2a

## Claims

1. A dilatation activatable tubular stent assembly having a proximal and a distal end and at least a first longitudinal section of first physical properties and at least a second longitudinal section of second physical properties, **characterized in that** said first and second stent sections (30; 20) of significantly different physical properties are arranged coaxially and overlapping in at least one selected portion of the length of the stent.

2. Stent assembly according to claim 1, **characterized in that** said first and second longitudinal sections are separate individual first and second stents (30; 20) inserted into each other.

3. Stent assembly according to claim 2, **characterized in that** the first stent section (30) is comprised of plastically deformable stent material.

4. Stent assembly according to claim 2, **characterized in that** the second stent section (20) is comprised of elastically deformable stent material.

5. Stent assembly according to any of the preceding claims, **characterized in that** the second stent (20) is protruding axially from at least one end (proximal and/or distal) of the first stent (30).

6. Stent assembly according to claim 5, **characterized in that** said at least one protruding end of the stent assembly is comprised of predominantly self-expanding elastically deformable stent material of shape-memory material forming a flaring end (22) of the protruding end of the stent defining a stent section lying essentially in a surface running perpendicular or obliquely to the longitudinal axis of the remainder of the stent assembly.

7. Stent assembly according to any of the preceding claims, **characterized in that** the second stent (20) is inserted inside the first stent (30).

8. Stent assembly according to any of the claims 1 to 6, **characterized in that** the second stent (20) is arranged on the outside surface of the first stent (30) and firmly connected thereto by glueing, welding, intertwining, or the like.

9. Stent assembly according to any of the preceding claims, **characterized in that** the elastically deformable stent material consists of a material significantly thinner than the material of the plastically deformable stent material.

10. Stent assembly according to any of the preceding claims, **characterized in that** the elastically deformable stent material consists of a mesh material with a gap size significantly smaller than that of the plastically deformable stent material.

11. Stent assembly according to any of preceding claims 1 to 7 and 9 and 10, **characterized in that** the inner stents (40; 41) and the outer stents (44; 45) of identical length before dilatation are physically connected to each other at one circumferential line (48; 49) along the axial length of both stents, whereby both stents exhibit different axial lengths after dilatation of the stent assembly (Fig 9c and 9d).

12. Stent assembly according to claim 11, **characterized in that** the circumferential connection line is located either centrally or next to the proximal or distal end of the stent assembly.

13. Stent assembly according to claim 11 or 12, **characterized in that** the two stents are separated additionally by a tubular sleeve of appropriate material, which sleeve is inserted radially between the two stents.
